# EUROPEAN PATENT APPLICATION

(11) **EP 4 497 418 A1**
(43) Date of publication of application: **29.01.2025**
(21) Application number: 24183780.6
(22) Date of filing: 21.06.2024
(51) Int. Cl.: A61F 2/95

(54) **ENDOVASCULAR IMPLANT DELIVERY SYSTEM WITH PREFERENTIAL ROTATION**

(30) Priority: 25.07.2023 US 202363528810 P; 29.05.2024 US 202418677355
(71) Applicant: Medtronic Vascular, Inc., Santa Rosa, CA 95403 (US)
(72) Inventor: FLORY, David E., Santa Rosa, 95403 (US); ZHOU, James J., Santa Rosa, 95403 (US)
(74) Representative: Zimmermann & Partner Patentanwälte mbB

(57) **Abstract**

An endovascular implant delivery system is configured to deploy an implant (e.g., a stent graft) having a branch within a peripheral/branch artery. The endovascular implant delivery system includes proximal and distal portions and a middle member extending therebetween. The middle member includes main and secondary channels to provide a preferential bending axis asymmetrically bisecting a radial cross section of the main and secondary channels. The middle member is configured to rotate against a first resistance in a first radial orientation in response to a first torque being applied to the middle member at the proximal portion and against a second resistance in a second radial orientation in response to a second torque being applied to the middle member at the proximal portion. The first resistance is less than the second resistance such that the first radial orientation is indicative of rotational alignment between the branch and a peripheral/branch artery.

## Description

### CROSS-REFERENCE TO RELATED APPLICATION

This application claims the benefit of U.S. provisional application Serial No. 63/528,810 filed July 25, 2023, the disclosure of which is hereby incorporated in its entirety by reference.

### TECHNICAL FIELD

The present disclosure relates to an endovascular implant delivery system with preferential rotation. The endovascular implant delivery system may be used in procedures to bridge aneurysms and other diseased blood vessels.

### BACKGROUND

Endovascular procedures are minimally invasive techniques to deliver clinical treatments in a patient's vasculature. One example of a clinical treatment used in an endovascular procedure is deployment of a stent graft. A stent graft is an implantable device made of a tube-shaped surgical graft material and an expanding (e.g., self-expanding) stent frame. The stent graft is placed inside a patient's vasculature (e.g., blood vessel) to bridge a diseased blood vessel segment (e.g., an aneurismal, dissected, or torn blood vessel segment), and thereby excluding hemodynamic pressures of blood flow from the diseased blood vessel segment.

The diseased blood vessel segment may extend into vasculature having blood vessel bifurcations or segments of the aorta from which smaller branch arteries extend. For example, thoracic aortic aneurysms may include aneurysms present in the aortic arch where branch arteries (e.g., the left subclavian, left common carotid, or the brachiocephalic arteries) emanate therefrom.

The endovascular procedure may be carried out with a modular stent graft system. The modular stent graft system may fail to align a branch or a coupling of a stent graft with a branch artery (e.g., the left subclavian, left common carotid, or the brachiocephalic arteries) or a peripheral artery (e.g., a common iliac artery, an external iliac artery, or an internal iliac artery). The result of this misalignment may be a lack of patency of the stent graft system, thereby potentially causing unacceptable occlusions in blood flow through the stent graft system.

### SUMMARY

In an embodiment, an endovascular implant delivery system is disclosed. The endovascular implant delivery system is configured to deploy an implant (e.g., a stent graft) having a branch (e.g., a branching stent graft or external mobile coupling) within a peripheral artery or a branch artery. The endovascular implant delivery system includes a proximal portion, a distal portion, and a middle member extending between the proximal portion and the distal portion. For example, the middle member may partially extend between the proximal portion and the distal portion (e.g., the middle member may extend to a position proximal where the stent graft is located within the delivery system). The middle member includes a main channel and a secondary channel formed therein to provide a preferential bending axis asymmetrically bisecting a radial cross section of the main and secondary channels. The middle member is configured to rotate against a first resistance in a first radial orientation in response to a first torque being applied to the middle member at the proximal portion and against a second resistance in a second radial orientation in response to a second torque being applied to the middle member at the proximal portion. The first resistance is less than the second resistance such that the first radial orientation is indicative of a rotational alignment between the branch of the implant and a peripheral artery or a branch artery.

The main channel may be configured to receive a main guidewire and the secondary channel may be configured to receive a secondary guidewire. The main channel and the secondary channel are configured to align the main guidewire and the secondary guidewire in a common plane when the middle member is in the first radial orientation. The common plane may be orthogonal a preferential bending axis of the middle member. In or more embodiments, the main guidewire and the secondary guidewire are not in the common plane when the middle member is in the second radial orientation. The main guidewire may be in a first plane and the secondary guidewire may be in a second plane when the middle member is in the second radial orientation. In one or more embodiments, the first and second planes are non-orthogonal a preferential bending axis of the middle member. The main channel may be a closed main guidewire channel. The secondary channel may open to an outer surface of the middle member. The middle member may be solid except for the main channel and the secondary channel.

In another embodiment, an endovascular implant delivery system is disclosed. The endovascular implant delivery system is configured to deploy an implant (e.g., a stent graft) having a branch (e.g., a branching stent graft or external mobile coupling) within a peripheral artery or a branch artery. The endovascular implant delivery system includes a proximal portion, a distal portion, and a middle member extending between the proximal portion and the distal portion. For example, the middle member may partially extend between the proximal portion and the distal portion (e.g., the middle member may extend to a position proximal where the stent graft is located within the delivery system). The middle member includes a main channel and a secondary channel formed therein to provide a preferential bending axis asymmetrically bisecting a radial cross section of the main and secondary channels. The middle member is configured to rotate in a first radial orientation in response to a first torque being applied to the middle member at the proximal portion and in a second radial orientation in response to a second torque being applied to the middle member at the proximal portion. The first torque is less than the second torque such that the first radial orientation is indicative of a rotational alignment between the branch of the implant and a peripheral artery or a branch artery.

In this embodiment, the middle member may have a first bending preference ratio in the first radial orientation and a second bending preference ratio in the second radial orientation. The first bending preference ratio is a first rotational distance compared to the first torque. The second bending preference ratio is a second rotational distance compared to the second torque. The first bending preference ratio is different than the second bending preference ratio. The first bending preference ratio may be greater than the second bending preference ratio. The first bending preference ratio may be less than 1.

In yet another embodiment, an endovascular implant delivery method for deploying an implant (e.g., a stent graft) having a branch (e.g., a branching stent graft or external mobile coupling) within a peripheral artery or a branch artery is disclosed. The method includes inserting an implant delivery device into a vasculature of a patient. The vasculature includes a peripheral artery or a branch artery. The implant delivery device has proximal and distal portions and a middle member extending therebetween. For example, the middle member may partially extend between the proximal portion and the distal portion (e.g., the middle member may extend to a position proximal where the stent graft is located within the delivery system). The middle member includes a main channel and a secondary channel formed therein to provide a preferential bending axis asymmetrically bisecting a radial cross section of the main and secondary channels. The method further includes rotating the middle member in a first radial orientation in response to a first torque being applied to the middle member at the proximal portion. The method also includes rotating the middle member in a second radial orientation in response to a second torque being applied to the middle member at the proximal portion. The rotating steps rotationally align the branch of the implant with the peripheral artery or the branch artery.

The first torque may be less than the second torque. The first rotating step may be carried out against a first resistance, and the second rotating step may be carried out against a second resistance. The second resistance may be greater than the first resistance. The method may further include receiving the first and second torques through an actuator situated at the proximal portion.

### BRIEF DESCRIPTION OF THE DRAWINGS

Figure 1 is a cross section view of an abdominal aorta depicting a side view of a branched stent graft within a first common iliac artery (CIA) and a first external iliac artery (EIA) and aligning with a first internal iliac artery (IIA).
Figure 2 is a cross section view of an aortic arch depicting a side view of a stent graft within the aorta and an external coupling extending into the left subclavian artery.
Figure 3A is an exploded view of an implant delivery system.
Figure 3B is an exploded view of an adapter assembly of the implant delivery system shown in Figure 3A.
Figure 3C is a cross section view of a stent graft cover including a main guidewire lumen situated within a stability lumen, a stent graft situated between the stent graft cover and the stability lumen.
Figure 4 is a cross section view of an abdominal aorta and depicts a side view of a portion of an implant delivery system including a main guidewire and a secondary guidewire.
Figure 5 is a cross section view of an aortic arch and depicts a side view of a portion of an implant delivery system including a main guidewire and a secondary guidewire.
Figure 6 is a radial cross section view of a middle member oriented in a first orientation.
Figure 7 is a radial cross section view of the middle member of Figure 6 in a second orientation.
Figure 8 is a radial cross section view of an alternative middle member according to a second embodiment.
Figure 9 is a radial cross section view of another alternative middle member according to a third embodiment.
Figure 10 is a radial cross section view of yet another alternative middle member according to a fourth embodiment.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of blood vessels such as the aorta, coronary, carotid, and renal arteries, the invention may also be used in any other body passageways (e.g., aortic valves, heart ventricles, and heart walls) where it is deemed useful.

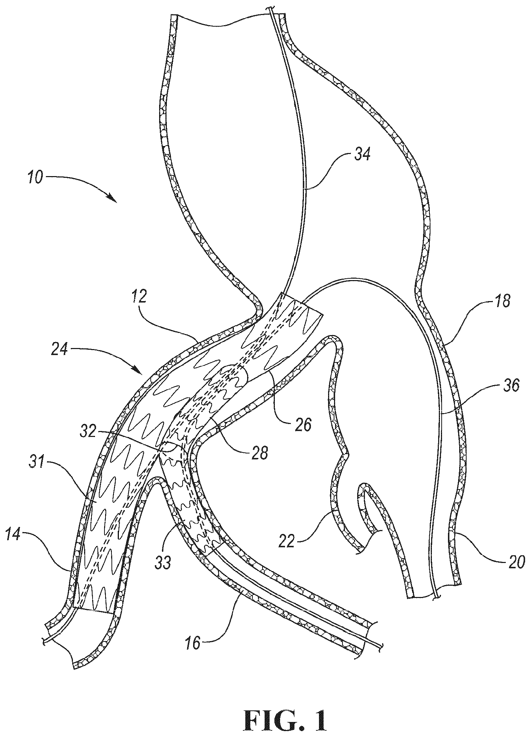

Figure 1 includes a cross section view of abdominal aorta 10. Abdominal aorta 10 branches into first common iliac artery (CIA) 12, which branches into first external iliac artery (EIA) 14 and first internal iliac artery (IIA) 16. Abdominal aorta 10 also branches into second CIA 18, which branches into second EIA 20 and second IIA 22.

Figure 1 also depicts a side view of bifurcated stent graft 24 extending within first CIA 12 and first EIA 14 and aligning with first IIA 16. Bifurcated stent graft 24 is configured to bridge an aneurysm in first CIA 12 while maintaining perfusion into first EIA 14 and first IIA 16. Bifurcated stent graft 24 includes proximal portion 26 branching into first branch limb 28 and second branch limb 31. First branch limb 28 forms first branch limb opening 32 configured to align with the ostia of first IIA 16. First branch limb extension 33 may be delivered to bifurcated stent graft 24 and deployed within first branch limb 28 as shown in Figure 1. First branch limb extension 33 extends within first IIA 16.

Figure 1 further depicts main guidewire 34 and secondary guidewire 36. Main guidewire 34 may be configured to deliver and deploy bifurcated stent graft 24 within first CIA 12 and first EIA 14. Second guidewire 36 may be configured to deliver and deploy first branch limb extension 33 within first branch limb 28 and first IIA 16.

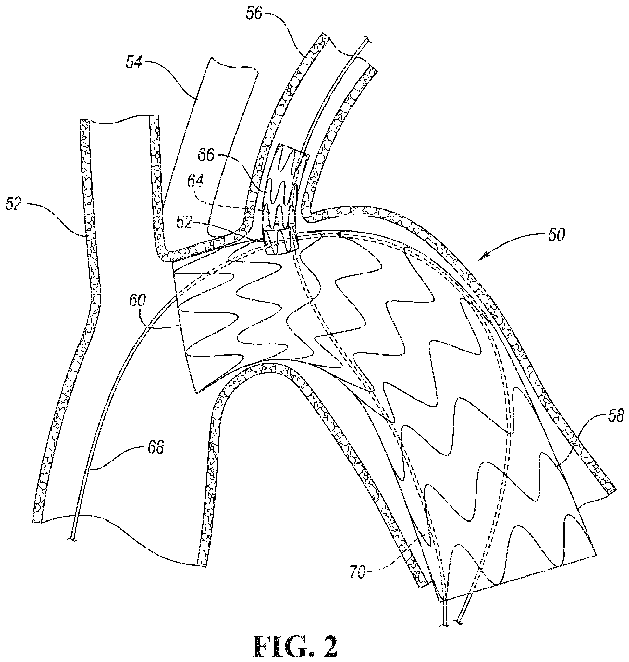

Figure 2 includes a cross section view of aortic arch 50. Aortic arch 50 branches into brachiocephalic artery 52, left common carotid artery 54, and left subclavian artery 56. Brachiocephalic artery 52, left common carotid artery 54, and left subclavian artery 56 may be referred to generally as side branch arteries.

Figure 2 also depicts a side view of stent graft 58 extending within aortic arch 50. Stent graft 58 includes proximal end 60 and a distal end (not shown). Stent graft 58 also includes an external coupling 62 (e.g., a mobile external coupling) configured to perfuse a branch artery otherwise excluded by stent graft 58. External coupling 62 forms external coupling opening 64. As shown in Figure 2, external coupling 62 and coupling opening 64 align with the ostia of left subclavian artery 56. Branch stent graft 66 may be delivered to stent graft 50 and deployed within external coupling 62 as shown in Figure 2. Branch stent graft 66 extends within left subclavian artery 56.

Figure 2 further depicts main guidewire 68 and secondary guidewire 70. Main guidewire 68 may be configured to deliver and deploy stent graft 58 within aortic arch 50. Secondary guidewire 70 may be configured to deliver and deploy branch stent graft 66 mobile external coupling 62 as shown in Figure 2.

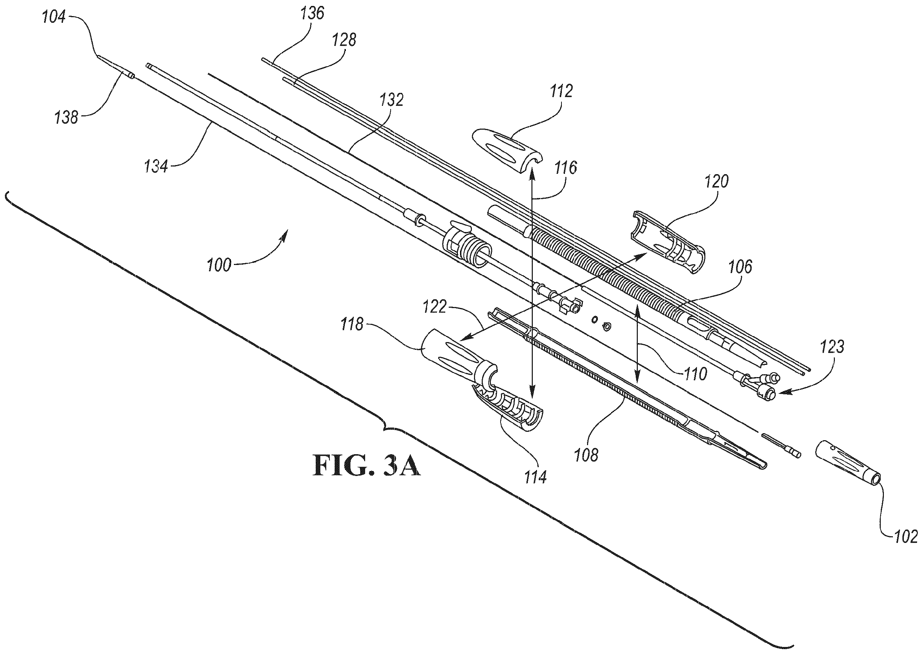

Figure 3A depicts an exploded view of implant delivery system 100 extending between proximal end 102 (e.g., closest to a clinician) and distal end 104 (e.g., furthest from the clinician). Implant delivery system 100 is configured to deliver and deploy an implant within a vasculature of a patient. Non-limiting examples of the implants that may be delivered using implant deliver system 100 include bifurcated stent graft 24, first branch limb extension 32, stent graft 58, and branch stent graft 66.

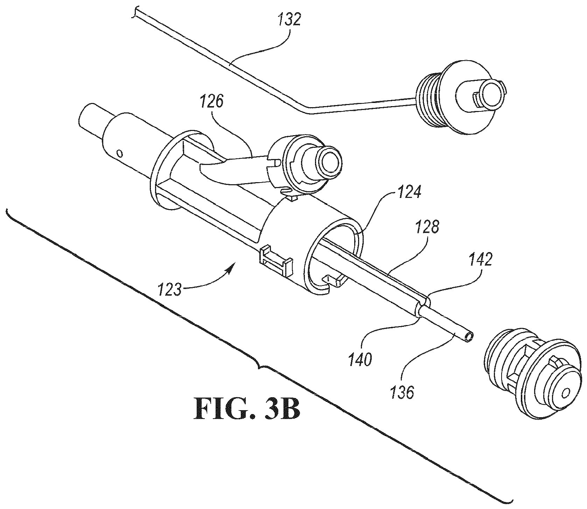

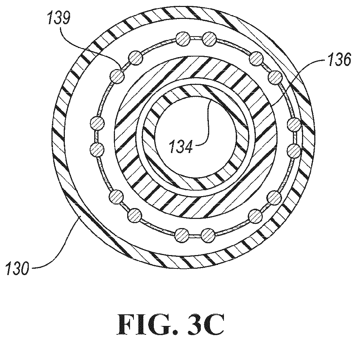

Implant delivery system 100 includes a threaded screw gear formed by bringing together and connecting (e.g., snap-fitting or otherwise assembling) first shell 106 and second shell 108. Arrowed line 110 represents bringing together first shell 106 and second shell 108 to form the assembled threaded screw gear. As depicted in Figure 3, the screw gear has threading. However, in other embodiments, the screw gear may not be threaded, thereby accommodating linear movement.

Implant delivery system 100 also includes a handle assembly. The handle assembly includes a front grip and an external slider. As shown in Figure 3, the front grip is formed by bringing together and connecting first shell 112 and second shell 114. Arrow 116 represents bringing together first shell 112 and second shell 114 to form the assembled front grip. The external slider is formed by bringing together and connecting first shell 118 and second shell 120. Arrowed line 122 represents bringing together first shell 118 and second shell 120 to form the assembled external slider.

The front grip may be fixed relative to the screw gear and the external slider may rotate about a threaded outer surface of the screw gear. During deployment of an implant (e.g., a stent graft), the external slider may be rotated to move toward proximal end 102. Since the external slider is operatively coupled to an implant cover (e.g., stent graft cover), the implant cover is retracted with the linear movement of the external slider, thereby permitting the stent graft to transition from a constrained position to an expanded position.

Implant delivery system 100 also includes adapter assembly 123, which includes main access port 124 and secondary access port 126. Adapter assembly 123 may be referred to as a Y-tube adapter assembly. Primary access port 124 includes an opening for insertion of middle member 128. As shown in Figure 3B, stability lumen 136 is inserted into middle member 128. Secondary guidewire lumen 132 is configured for insertion into secondary access port 126 and middle member 128.

In one or more embodiments, an adapter assembly (e.g., adapter assembly 123) serves as a junction to permit a main guidewire lumen 134 (e.g., to receive a main guidewire) for tracking an implant (e.g., stent graft) through the vasculature of a patient and a secondary guidewire lumen 132 (e.g. to receive a secondary guidewire) for internal limb cannulation (e.g., cannulation of first branch limb extension 32 or branch stent graft 66) where the main guidewire and secondary guidewire extend within a stent graft cover and exit through separate access ports (e.g., primary access port 124 and secondary access port 126, respectively).

As depicted in Figure 3C, stent graft cover 130 includes main guidewire lumen 134 and stability lumen 136. Main guidewire lumen 134 is situated within stability lumen 136. Tapered tip 138 (shown in Figure 3B) serves as an introducer tip configured to dilate the vasculature up to an outer diameter of implant delivery system, thereby reducing vessel trauma. The tapered tip may be configured to track over the main guidewire and to provide access to a secondary guidewire.

During implant delivery, stent graft 139 is loaded between stent graft cover 130 and stability lumen 136. Stability lumen 136 is configured to provide a stabilizing friction force to resist shifting of stent graft 139 during deployment. Stability lumen 136 may grip onto the inner surface of stent graft 139 and provide axial stiffness to resist friction of stent graft 139. Stability lumen 136 may also be configured to maintain attachment to a distal end of stent graft 139 during delivery of stent graft 139.

As shown in Figure 3B, middle member 128 includes main channel 140 and secondary channel 142. While main channel 140 is a closed loop, secondary channel 142 has an open trench in the embodiment shown. Stability lumen 136 extends within main channel 140. Middle member 128 is configured to support stability lumen 136 during delivery and deployment of stent graft 139 to prevent kinks in stent graft cover 130. The middle member may also be configured to provide columnar support to implant delivery system 100 during delivery and deployment of stent graft 139. The middle member may also be configured to resist migration of stent graft 139 within stent graft cover 130 during deployment of stent graft 139.

In one or more embodiments, the middle member is configured to aid in the rotational and axial alignment of an implant within a branch or peripheral artery. The rotational alignment may refer to the alignment of an implant branch (e.g., a branch limb extension, an external coupling, a mobile external coupling, or an opening/fenestration (formed during manufacturing or in-situ)) such that the longitudinal axis of the implant branch aligns with the longitudinal axis of a branch or peripheral artery or ostia thereof. The axial alignment may refer to the alignment of the implant branch such that the implant branch faces or extends into the branch or peripheral artery upon deployment of the implant branch. Rotational and axial alignment serve to provide the implant access to a branch or peripheral artery. Rotational and axial alignment also provides patency to the implant, including its branches to create unobstructed blood flow through the arteries in which the implant is deployed.

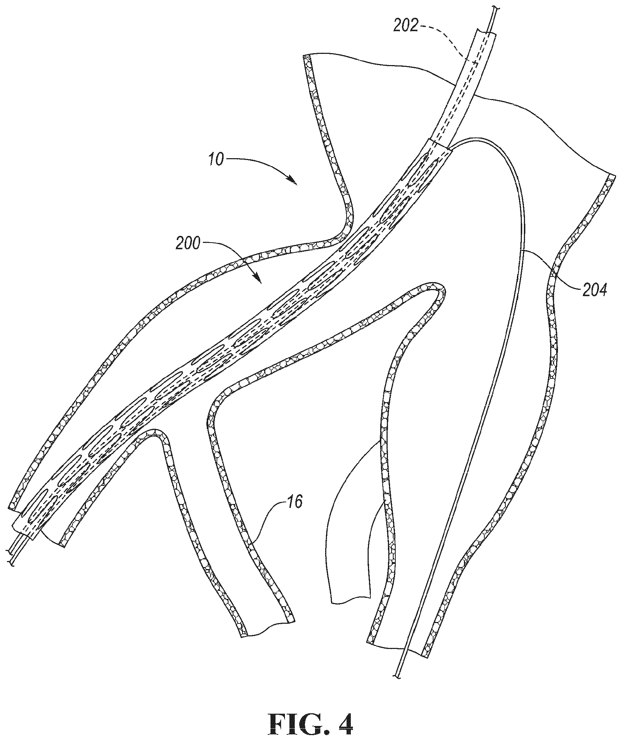

Figure 4 is a cross section view of abdominal aorta 10 and depicts a side view of a portion of implant delivery system 200 including main guidewire 202 and secondary guidewire 204. Deployment of an implant branch within first IIA 16 may have a preferred rotational alignment. The preferred rotational alignment may also be applied to other peripheral arteries such as second IIA 22. The preferred rotational alignment may extend along the cross section of first IIA 16 as depicted in Figure 4. The cross section of Figure 4 longitudinally bisects first IIA 16. An aligned deployed implant branch can occupy first IIA 16 while reducing interference with the ostia of first IIA 16 and the surrounding vessel walls and structure. This preferred rotational alignment is configured to provide patency to the implant branch.

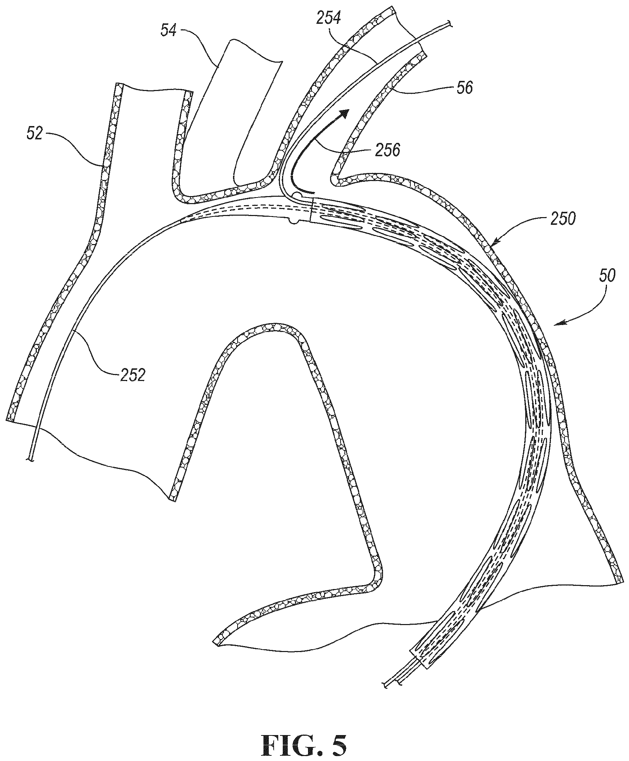

Figure 5 is a cross section view of aortic arch 50 and depicts a side view of a portion of implant delivery system 250 including main guidewire 252 and secondary guidewire 254. Figure 5 also includes arrow 256 depicting a preferred rotational alignment for deploying an implant branch within left subclavian artery 56. The preferred rotational alignment may also be applied to brachiocephalic artery 52 and left common carotid artery 54. Arrow 256 extends along the cross section of left subclavian artery 56 as depicted in Figure 5. The cross section of Figure 5 longitudinally bisects left subclavian artery 56. As arrow 206 aligns with the longitudinal axis of the implant branch once deployed, the deployed implant branch can occupy left subclavian artery 56 while reducing interference with the ostia of left subclavian artery 56 and the surrounding vessel walls and structure. This preferred rotational alignment is configured to provide patency to the implant branch.

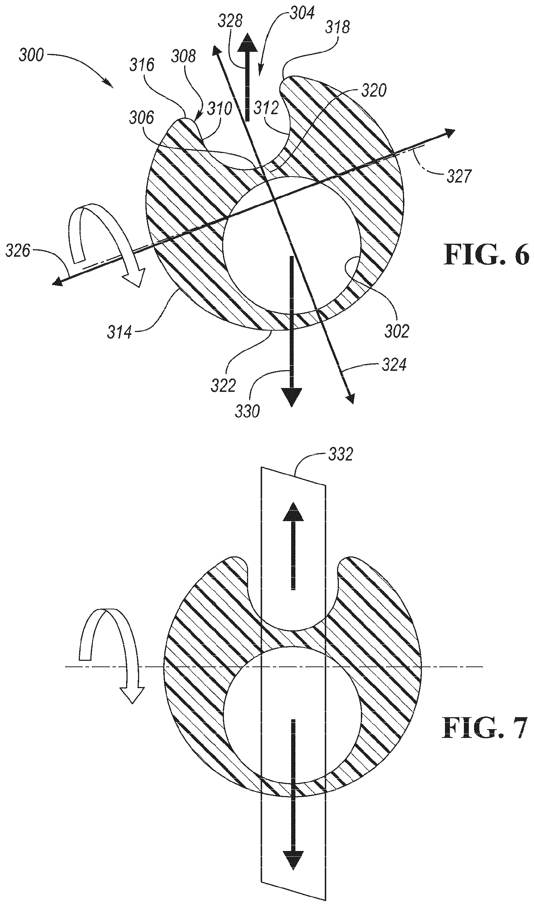

Figure 6 is a radial cross section view of middle member 300 oriented in a first radial orientation. Figure 7 is a radial cross section view of middle member 300 in a second radial orientation. As shown in the radial cross section views, middle member 300 has a cylindrical shape. The average diameter of a cross section of middle member 300 may be one of the following values or in a range of any two of the following values: 0.178, 0.179, 0.180, 0.181, and 0.182 inches. The cross-sectional diameter of middle member 300 may be consistent throughout the length of middle member 300 within a tolerance (e.g., a tolerance of ± 0.002 inches).

Middle member 300 is formed of a solid material (e.g., a polymeric material) except for main closed channel 302 and secondary open channel 304. In one or more embodiments, main closed channel 302 and secondary open channel 304 run the entire length of middle member 300.

Main closed channel 302 has a cylindrical shape. The average diameter of a cross section of main closed channel 302 may be one of the following values or in a range of any two of the following values: 0.094, 0.095, 0.096, 0.097, and 0.098 inches. The cross-sectional diameter of main closed channel 302 may be consistent throughout the length of main closed channel 302 within a tolerance. Main closed channel 302 may be configured to contain at least a portion of stability lumen 136 and a tapered tip lumen.

Secondary open channel 304 includes inner portion 306 and outer portion 308. Inner portion 306 has an arcuate shape and an arcuate-shaped radial cross section. The diameter of the circular-shaped radial cross section may be one of the following values or in a range of any two of the following values: 0.060, 0.061, 0.062, 0.063, and 0.064 inches. As shown in Figures 6 and 7, outer portion 308 includes first wall 310 and second wall 312 connected to outer surface 314 of middle member 300 through first curved surface 316 and second curved surface 318, respectively. First and second walls 310 and 312 may be planar-shaped within a tolerance. The profile of secondary open channel 304 may be consistent throughout the length of secondary open channel 304 within a tolerance. Secondary open channel 304 is configured to contain at least a portion of secondary guidewire lumen 132.

The center of middle member 300, main closed channel 302 and/or secondary open channel 304 (e.g., inner portion 306) may be aligned with second axis 324 within a tolerance. Main closed channel 302 and secondary open channel 304 may be spaced apart from each other by inner wall thickness 320. The inner wall thickness 320 may be any of the following values or in a range of any two of the following values: 0.007, 0.008, and 0.009 inches. Main closed channel 302 may be spaced apart from outer surface 314 by outer wall thickness 322. Outer wall thickness 322 may be any of the following values or in a range of any two of the following values: 0.007, 0.008, and 0.009 inches.

Middle member 300 includes first axis 324 and second axis 326, which is perpendicular to first axis 324. Middle member 300 is symmetrical about first axis 324 and is asymmetrical about second axis 326. The asymmetrical characteristic about second axis 326 is configured to provide middle member 300 a preferential bending axis 327 (e.g., a minimum stiffness bending axis). Preferential bending axis 327 is configured to provide differential axial rotation of middle member 300. For instance, axial rotation of middle member 300 when middle member 300 is in the first orientation shown in Figure 6 is more difficult than when middle member 300 is in the second orientation shown in Figure 7. In the first unpreferred orientation, secondary guidewire tension 328 from a secondary guidewire (e.g., secondary guidewire 204 or 254) is not aligned in a common plane with main guidewire tension 330 from a main guidewire (e.g., main guidewire 202 or 252). In one or more embodiments, the common plane is orthogonal preferential bending axis 327 (e.g., common plane 332 as shown in Figures 6 and 7). In the second preferred orientation, main guidewire tension 330 is aligned in a common plane with secondary tension 328 (e.g., common plane 332 as shown in Figure 7).

A clinician using an implant delivery system receives tactile feedback indicative of requiring the application of additional twisting force from a proximal portion of the implant delivery system to rotate middle member 300. This bending resistance may be characterized as a bending preference ratio (e.g., the amount of rotational movement of the middle member compared to the amount of twisting force to rotate the middle member). A ratio of 1 indicates no preference to bending. A ratio of 1 may be beneficial to extend the middle member (and the rest of the implant delivery system) through a tortious vasculature of a patient. A ratio of 1 may indicate an isotropic condition. In one or more embodiments, the bending preference ratio of middle member 300 may be less than 1. In one or more embodiments, the bending preference ratio of middle member 300 may be any of the following values or in a range of any two of the following values: 0.6, 0.65, 0.7, 0.75, 0.8, 0.85, 0.9, 0.95, thereby imparting a bending preference depending on the rotational alignment of the first and second guidewires.

The middle member of one or more embodiments is configured to provide differential tactile feedback with rotational alignment. In one or more embodiments, rotation becomes easier as orientation becomes closer to an ideal alignment, thereby permitting precise fine tuning of the implant rotational alignment. In one or more embodiments, rotation becomes more difficult the farther alignment is from the ideal alignment, thereby providing a tactile indication that the optimum alignment is in the opposite direction from the direction of rotation. As an addition benefit of one or more embodiments, the implant delivery system is less likely to move rotationally when other implants are being connected due to preferential alignment with a desired orientation.

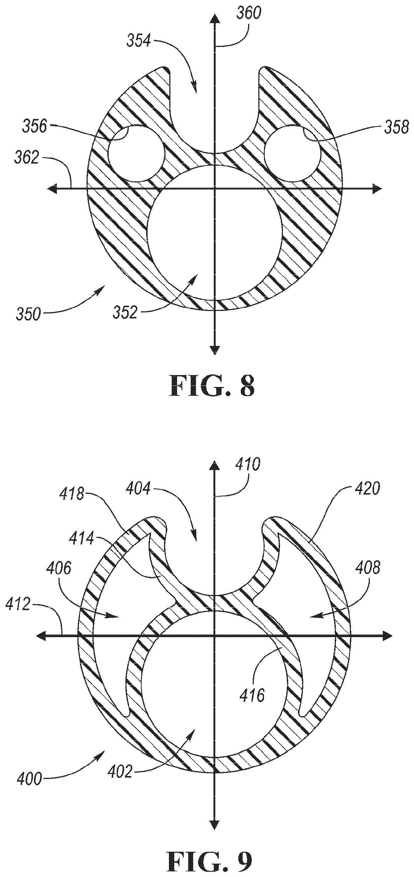

Figure 8 is a radial cross section view of middle member 350 according to a second embodiment. Middle member 350 may have an average diameter as set forth above. Middle member 350 is formed of a solid material (e.g., a polymeric material) except for main closed channel 352, secondary open channel 354, and first and second offset channels 356 and 358. Main closed channel 352 and secondary open channel 354 may have the diameters and other dimensions as set forth above. First and second offset channels 356 and 358 may have a diameter independently selected from any of the following values or in a range of any two of the following values: 0.038, 0.039, 0.040, 0.041, and 0.042 inches. Middle member 350 is symmetrical about first axis 360 and is asymmetrical about second axis 362 to provide a preferential bending axis to middle member 350 in accordance with one or more embodiments.

Figure 9 is a radial cross section view of middle member 400 according to a third embodiment. Middle member 400 may have an average diameter as set forth above. The walls of middle member 400 may be formed using an extrusion process. The walls of middle member 400 form main closed channel 402, secondary open channel 404, and first and second wings 406 and 408. Wings 406 and 408 may be closed channels (e.g., hollow cavities). Main closed channel 402 and secondary open channel 404 may have the diameters and other dimensions as set forth above. Middle member 400 is symmetrical about first axis 410 and is asymmetrical about second axis 412 to provide a preferential bending axis to middle member 400 in accordance with one or more embodiments. Inner wall 414 defines secondary open channel 404. Inner wall 416 partially defines main closed channel 402. A portion of inner wall 414 and inner wall 416 and outer wall 418 define wing 406. A portion of inner wall 414 and inner wall 416 and outer wall 420 define wing 408.

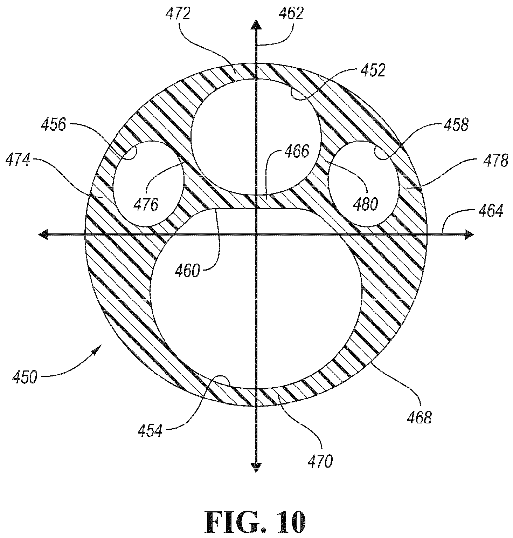

Figure 10 is a radial cross section view of middle member 450 according to a fourth embodiment. Middle member 450 is formed of a solid material (e.g., a polymeric material) except for upper closed channel 452, lower closed channel 454, and first and second offset channels 456 and 458. In one or more embodiments, upper closed channel 452, lower closed channel 454, and first and second offset channels 456 and 458 run the entire length of middle member 450.

Upper closed channel 452 has a cylindrical shape (e.g., a generally cylindrical shape). The cross-sectional diameter of upper closed channel 452 may be consistent throughout the length of upper closed channel 452 within a tolerance. Upper closed channel 452 is configured to contain at least a portion of a secondary guidewire lumen.

Lower closed channel 454 has a generally cylindrical shape. As shown in Figure 9, lower closed channel 454 includes a generally flat upper surface 460. The cross-sectional diameter of lower closed channel 454 may be consistent throughout the length of lower closed channel 454. Lower closed channel 454 may be configured to contain at least a portion of a stability lumen and a tapered tip lumen.

First and second offset channels 456 and 458 may have a diameter independently selected from any of the values or within a range of any two of the values set forth above. Middle member 450 is symmetrical about first axis 462 and is asymmetrical about second axis 464 to provide a preferential bending axis to middle member 450 in accordance with one or more embodiments.

Upper closed channel 452 and lower closed channel 454 may be spaced apart from each other by inner wall thickness 466. Lower closed channel 454 may be spaced apart from outer surface 468 by lower outer wall thickness 470. Upper closed channel 452 may be spaced apart from outer surface 468 by upper outer wall thickness 472. First offset channel 456 may be spaced apart from outer surface 468 by first side outer wall thickness 474 and may be spaced apart from upper closed channel 452 by second inner wall thickness 476. Second offset channel 458 may be spaced apart from outer surface 468 by second side outer wall thickness 478 and may be spaced apart from upper closed channel 452 by third inner wall thickness 480.

The middle members of one or more embodiments disclosed herein interact with delivery systems in which such middle member is implemented (e.g., bifurcated or branched implant delivery systems within the abdominal aorta or the aortic arch) and the vasculature of the patient to provide preferential rotational alignment for deploying an implant branch within a branch or peripheral vessel. In one or more embodiments, the orientation of the middle member, a side branch of a stent graft, and the delivery system may be collectively configured such that a minimum bending stiffness occurs when the side branch is opposite the ostia of the branch or peripheral vessel. The delivery system including the middle member may be configured to provide feedback to the clinician that the minimum bending stiffness corresponds to alignment with the ostia. This configuration may be implemented by having external markers on a handle of the delivery system for alignment or keying an orientation of the middle member to a Y-adapter or other component of the delivery system. The configuration may differ depending on the procedure (e.g., delivery of a bifurcated stent graft within the abdominal aorta or delivery of a branched stent graft in the aortic arch). The configuration may also differ depending on the type of peripheral or branch vessel intended for the procedure.

Further disclosed herein is an endovascular implant delivery system. The endovascular implant delivery system is configured to deploy an implant (e.g., a stent graft) having a branch within a peripheral/branch artery. The endovascular implant delivery system includes proximal and distal portions and a middle member extending therebetween. The middle member includes main and secondary channels to provide a preferential bending axis asymmetrically bisecting a radial cross section of the main and secondary channels. The middle member is configured to rotate against a first resistance in a first radial orientation in response to a first torque being applied to the middle member at the proximal portion and against a second resistance in a second radial orientation in response to a second torque being applied to the middle member at the proximal portion. The first resistance is less than the second resistance such that the first radial orientation is indicative of rotational alignment between the branch and a peripheral/branch artery.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

Further disclosed herein is the subject matter of the following clauses:
1. An endovascular implant delivery system for deploying an implant having a branch, the endovascular implant delivery system comprising:
   a proximal portion;
   a distal portion; and
   a middle member extending between the proximal portion and the distal portion, the middle member includes a main channel and a secondary channel formed therein to provide a preferential bending axis asymmetrically bisecting a radial cross section of the main and secondary channels, the middle member is configured to rotate against a first resistance in a first radial orientation in response to a first torque being applied to the middle member at the proximal portion and against a second resistance in a second radial orientation in response to a second torque being applied to the middle member at the proximal portion, the first resistance is less than the second resistance such that the first radial orientation is indicative of a rotational alignment between the branch of the implant and a peripheral artery or a branch artery.
2. The endovascular implant delivery system of clause 1, wherein the main channel is a configured to receive a main guidewire and the secondary channel is configured to receive a secondary guidewire.
3. The endovascular implant delivery system of clause 2, wherein the main channel and the secondary channel are configured to align the main guidewire and the secondary guidewire in a common plane when the middle member is in the first radial orientation.
4. The endovascular implant delivery system of clause 3, wherein the common plane is orthogonal a preferential bending axis of the middle member.
5. The endovascular implant delivery system of clause 3 or 4, wherein the main guidewire and the secondary guidewire are not in the common plane when the middle member is in the second radial orientation.
6. The endovascular implant delivery system of clause 3 or any one of clauses 2 to 5, wherein the main guidewire is in a first plane and the secondary guidewire is in a second plane when the middle member is in the second radial orientation.
7. The endovascular implant delivery system of clause 6, wherein the first and second planes are non-orthogonal a preferential bending axis of the middle member.
8. The endovascular implant delivery system of clause 1 or of any preceding clause, wherein the main channel is a closed main guidewire channel.
9. The endovascular implant delivery system of clause 1 or of any preceding clause, wherein the secondary channel opens to an outer surface of the middle member.
10. The endovascular implant delivery system of clause 1 or of any preceding clause, wherein the middle member extends between proximal end and the implant.
11. An endovascular implant delivery system for deploying an implant having a branch, the endovascular implant delivery system comprising:
   a proximal portion;
   a distal portion; and
   a middle member extending between the proximal portion and the distal portion, the middle member includes a main channel and a secondary channel formed therein to provide a preferential bending axis asymmetrically bisecting a radial cross section of the main and secondary channels, the middle member is configured to rotate in a first radial orientation in response to a first torque being applied to the middle member at the proximal portion and in a second radial orientation in response to a second torque being applied to the middle member at the proximal portion, the first torque is less than the second torque such that the first radial orientation is indicative of a rotational alignment between the branch of the implant and a peripheral artery or a branch artery.
12. The endovascular implant delivery system of clause 11, wherein the middle member has a first bending preference ratio in the first radial orientation and a second bending preference ratio in the second radial orientation, the first bending preference ratio is a first rotational distance compared to the first torque, the second bending preference ratio is a second rotational distance compared to the second torque, the first bending preference ratio is different than the second bending preference ratio.
13. The endovascular implant system of clause 12, wherein the first bending preference ratio is greater than the second bending preference ratio.
14. The endovascular implant system of clause 12 or 13, wherein the first bending preference ratio is less than 1.
15. The endovascular implant system of clause 13 or any one of clauses 12 to 14, wherein the second bending preference ratio is less than 1.
16. An endovascular implant delivery method for deploying an implant having a branch, the method comprising:
   inserting an implant delivery device into a vasculature of a patient, the vasculature including a peripheral artery or a branch artery, the implant delivery device having proximal and distal portions and a middle member extending therebetween, the middle member including a main channel and a secondary channel formed therein to provide a preferential bending axis asymmetrically bisecting a radial cross section of the main and secondary channels;
   rotating the middle member in a first radial orientation in response to a first torque being applied to the middle member at the proximal portion; and
   rotating the middle member in a second radial orientation in response to a second torque being applied to the middle member at the proximal portion,
   the rotating steps rotationally aligning the branch of the implant with the peripheral artery or the branch artery.
17. The method of clause 16, wherein the first torque is less than the second torque.
18. The method of clause 16, wherein the first rotating step is carried out against a first resistance, and the second rotating step is carried out against a second resistance.
19. The method of clause 18, wherein the second resistance is greater than the first resistance.
20. The method of clause 16 further comprising receiving the first and second torques through an actuator situated at the proximal portion.

## Claims

1. An endovascular implant delivery system for deploying an implant having a branch,
the endovascular implant delivery system comprising:
a proximal portion;
a distal portion; and
a middle member extending between the proximal portion and the distal portion, the middle member includes a main channel and a secondary channel formed therein to provide a preferential bending axis asymmetrically bisecting a radial cross section of the main and secondary channels, the middle member is configured to rotate against a first resistance in a first radial orientation in response to a first torque being applied to the middle member at the proximal portion and against a second resistance in a second radial orientation in response to a second torque being applied to the middle member at the proximal portion, the first resistance is less than the second resistance such that the first radial orientation is indicative of a rotational alignment between the branch of the implant and a peripheral artery or a branch artery.

2. The endovascular implant delivery system of claim 1, wherein the main channel is a configured to receive a main guidewire and the secondary channel is configured to receive a secondary guidewire.

3. The endovascular implant delivery system of claim 2, wherein the main channel and the secondary channel are configured to align the main guidewire and the secondary guidewire in a common plane when the middle member is in the first radial orientation.

4. The endovascular implant delivery system of claim 3, wherein the common plane is orthogonal a preferential bending axis of the middle member.

5. The endovascular implant delivery system of claim 3 or 4, wherein the main guidewire and the secondary guidewire are not in the common plane when the middle member is in the second radial orientation.

6. The endovascular implant delivery system of any one of claims 2 to 5, wherein the main guidewire is in a first plane and the secondary guidewire is in a second plane when the middle member is in the second radial orientation.

7. The endovascular implant delivery system of claim 6, wherein the first and second planes are non-orthogonal a preferential bending axis of the middle member.

8. The endovascular implant delivery system of any preceding claim, wherein the main channel is a closed main guidewire channel.

9. The endovascular implant delivery system of any preceding claim, wherein the secondary channel opens to an outer surface of the middle member.

10. The endovascular implant delivery system of any preceding claim, wherein the middle member extends between proximal end and the implant.

11. An endovascular implant delivery system for deploying an implant having a branch, the endovascular implant delivery system comprising:
a proximal portion;
a distal portion; and
a middle member extending between the proximal portion and the distal portion, the middle member includes a main channel and a secondary channel formed therein to provide a preferential bending axis asymmetrically bisecting a radial cross section of the main and secondary channels, the middle member is configured to rotate in a first radial orientation in response to a first torque being applied to the middle member at the proximal portion and in a second radial orientation in response to a second torque being applied to the middle member at the proximal portion, the first torque is less than the second torque such that the first radial orientation is indicative of a rotational alignment between the branch of the implant and a peripheral artery or a branch artery.

12. The endovascular implant delivery system of claim 11, wherein the middle member has a first bending preference ratio in the first radial orientation and a second bending preference ratio in the second radial orientation, the first bending preference ratio is a first rotational distance compared to the first torque, the second bending preference ratio is a second rotational distance compared to the second torque, the first bending preference ratio is different than the second bending preference ratio.

13. The endovascular implant system of claim 12, wherein the first bending preference ratio is greater than the second bending preference ratio.

14. The endovascular implant system of claim 12 or 13, wherein the first bending preference ratio is less than 1.

15. The endovascular implant system of any one of claims 12 to 14, wherein the second bending preference ratio is less than 1.
